# EUROPEAN PATENT APPLICATION

(11) **EP 4 524 552 A1**
(43) Date of publication of application: **19.03.2025**
(21) Application number: 22941738.1
(22) Date of filing: 13.05.2022
(51) Int. Cl.: G01N 21/76, C12M 1/34

(54) **OPTICAL MEASUREMENT DEVICE**

(71) Applicant: HITACHI HIGH-TECH CORPORATION, Tokyo 105-6409 (JP)
(72) Inventor: ISHIZUKA Junji, Tokyo 105-6409 (JP)
(74) Representative: Strehl & Partner mbB
(86) International application number: PCT/JP2022/020271
(87) International publication number: WO 2023/218662

(57) **Abstract**

The present invention realizes an optical measurement device 202 that suppresses the influence of thermal radiation on sample temperature adjustment mechanisms 403, 404, 405 for adjusting the temperature inside a dark box 207 in which a sample is arranged, is able to efficiently maintain a low temperature inside the dark box 207 while maintaining a light-blocking effect, and is able to detect bioluminescence/chemiluminescence of a substance with high sensitivity and high precision. This optical measurement device 202 comprises: a detector 406 that has a light-receiving unit 407 that receives light generated by a sample tube 803 arranged inside the dark box 207, and detects the generated light; a temperature adjustment block 401 that is arranged in the periphery of the detector 406, a portion of the temperature adjustment block 401 being arranged inside the dark box 207 and the other portion thereof being arranged outside the dark box 207; and the sample temperature adjustment mechanisms 403, 404, 405 that are connected to the portion of the temperature adjustment block 401 arranged outside of the dark box 207.

## Description

### Technical Field

The present invention relates to an optical measurement device for detecting chemiluminescence and bioluminescence of a substance contained in a liquid sample with high sensitivity and high precision.

### Background Art

In a pharmaceutical plant or a beverage plant, a product is manufactured in a manufacturing facility in which a sterile environment is provided.

In the related art, a culture method has been adopted in a microorganism test for ensuring sterility of the manufacturing facility and the product. The culture method is a method in which a specimen to which a culture medium is added is cultured for several to 14 days during a high-temperature period, and the number of colonies of grown bacteria is visually counted. Therefore, it takes time to obtain a test result, and it is necessary to wait for the test result before the product can be shipped.

In view of such a background, it is desired to develop a rapid microorganism testing method for rapidly determining sterility.

One of rapid and simple microorganism testing methods is an adenosine triphosphate (ATP) bioluminescence method (hereinafter referred to as an ATP method). The ATP method is a method of performing measurement by converting ATP present within bacteria into light using a luminescence reaction of a firefly.

More specifically, luciferin and an ATP molecule are incorporated into luciferase, and a luminescence amount when oxidized luciferin (oxyluciferin) transitions from an excited state to a ground state as the ATP is consumed is measured.

At this time, a photon is generated by the consumption of the ATP molecule. The number of the photons generated is proportional to the number of the ATP. The ATP molecule is present in viable bacteria as an energy source, and a total number of the viable bacteria can be estimated by measuring the luminescence amount produced by the ATP in the specimen.

A luciferin-luciferase reaction has the most excellent quantum efficiency (ΦBL: ≈ 0.5) in bioluminescence and chemiluminescence, and thus one cell can be detected as several hundred thousand of photons.

Accordingly, the ATP method is a method capable of detecting light corresponding to one cell in principle.

However, in order to detect an extremely small amount (a level of several) of bacteria mixed in a specimen with high sensitivity and high precision, it is necessary to increase sensitivity of a luminescence measurement device.

A luminescence coefficient according to the ATP method is measured using a reagent kit from a certain manufacturer, and a measurement procedure is briefly described as follows.
1) An ATP eliminating solution is dispensed into a sample solution to eliminate ATP in dead bacteria other than viable bacteria and the sample solution.
2) An ATP extraction solution is dispensed into the sample solution to extract ATP from the viable bacteria.
3) A luminescent reagent is dispensed into the sample solution.
4) The luminescence amount is measured.

In order to increase sensitivity of a luminescence measurement, it is necessary to maintain a temperature of a detector at a low temperature, to reduce noise of a measurement device, and to eliminate influence of ambient light by making a portion holding the measurement device as a light-blocked space.

Since the luminescence occurs immediately when the luminescent reagent is dispensed into the sample container, in order to measure an accurate ATP amount, it is desirable to perform the process 3) and the subsequent processes in a state in which the sample is in the detector.

Although it is general to eliminate influence of ambient light by covering a photodetector and a sample container with a light-blocking structure, it is also effective to locally block light to a luminescence measurement device, as in the case with a light meter and in other cases in which an openable shutter is mounted in front of a light receiving surface of the photodetector, and the like.

Meanwhile, it is also necessary to prevent contamination such as ATP and bacteria present in the environment, microorganisms carried by a person, and dust adsorbing ATP. It is important to automate dispensing of a reagent, sample conveyance to a measurement position, and the like in a device, to reduce an operation performed by a person as much as possible, and to eliminate circulation of air and the like in a chamber as much as possible.

PTL 1 describes a technique for preventing external light from entering a luminescence detection unit when a luminescence measurement is not performed to prevent deterioration by providing a shutter that is normally closed and is opened at a time of the luminescence measurement.

PTL 2 describes a technique in which, in preparation for a measurement, a shutter unit is set to be in a closed state to block stray light from entering a photodetector, and in a measurement state, the shutter unit is set to be in an open state.

### Citation List

### Patent Literature

PTL 1: JPH07-83831A
PTL 2: JP2008-268019A

### Summary of Invention

### Technical Problem

In an analyzer that counts the number of photons using a photodetector, it is necessary to suppress a dark level of a light-receiving unit to a certain level or less in order to increase detection sensitivity.

Generally, in order to suppress the dark level of the photodetector, it is necessary to arrange the light-receiving unit in a dark box structure and to adjust a temperature to a low temperature.

In the case in which a temperature adjustment mechanism of the photodetector is arranged in the dark box structure, the temperature in the dark box structure is increased due to heat dissipation accompanying temperature adjustment, and efficiency of an enzyme reaction is reduced.

Techniques described in PTL 1 and PTL 2 teach light blocking, but do not describe maintaining a low temperature inside a dark box, and do not acknowledge or take into consideration the problem that the efficiency of the enzyme reaction is reduced due to the heat dissipation from the temperature adjustment mechanism.

An object of the invention is to implement an optical measurement device capable of reducing influence of heat dissipation from a temperature adjustment mechanism that adjusts a temperature in a dark box in which a sample is arranged, efficiently maintaining a low temperature inside the dark box while maintaining a light-blocking effect, and detecting bioluminescence and chemiluminescence of a substance with high sensitivity and high precision.

### Solution to Problem

In order to achieve the above object, the invention is formed as follows.

An optical measurement device includes: a detector that includes a light-receiving unit configured to receive light generated from a sample tube arranged in a dark box and that is configured to detect the generated light; a temperature adjustment block arranged around the detector; and a sample temperature adjustment mechanism connected to the temperature adjustment block. A part of the temperature adjustment block is arranged in the dark box and the other part of the temperature adjustment block is arranged outside the dark box, and the sample temperature adjustment mechanism is connected to a portion of the temperature adjustment block arranged outside the dark box.

### Advantageous Effects of Invention

According to the invention, it is possible to implement an optical measurement device capable of reducing influence of heat dissipation from a temperature adjustment mechanism that adjusts a temperature in a dark box in which a sample is arranged, efficiently maintaining a low temperature inside the dark box while maintaining a light-blocking effect, and detecting bioluminescence and chemiluminescence of a substance with high sensitivity and high precision.

### Brief Description of Drawings

[FIG. 1] FIG. 1 is a diagram showing an overall configuration of a rapid microorganism testing device according to an embodiment.
[FIG. 2] FIG. 2 is a diagram showing an overall configuration of the rapid microorganism testing device according to the embodiment.
[FIG. 3] FIG. 3 is a diagram showing a schematic configuration of the inside of the rapid microorganism testing device according to the embodiment.
[FIG. 4] FIG. 4 is a diagram showing a schematic configuration of a photodetector unit according to the embodiment.
[FIG. 5] FIG. 5 is a schematic configuration diagram of a sample tube conveying mechanism.
[FIG. 6] FIG. 6 is a diagram showing a schematic configuration of a reagent dispenser.
[FIG. 7] FIG. 7 is a view showing a schematic configuration of a reagent rack.
[FIG. 8] FIG. 8 is a diagram schematically showing a light-blocking structure of the photodetector unit.
[FIG. 9] FIG. 9 is a diagram showing a schematic configuration of a light-receiving unit of the photodetector unit according to the embodiment.
[FIG. 10] FIG. 10 is a flowchart showing a typical example of a luminescence measurement in the embodiment.
[FIG. 11] FIG. 11 is a diagram showing a schematic configuration of a modification of the photodetector unit according to the embodiment.

### Description of Embodiments

Hereinafter, an embodiment of the invention will be described with reference to the accompanying drawings.

However, the present embodiment is merely an example for implementing the invention, and does not limit the invention. In the drawings, the same reference numerals are given to the same configurations. In the following description of the embodiment, a term "sample" is synonymous with a term "sample."

### Embodiment

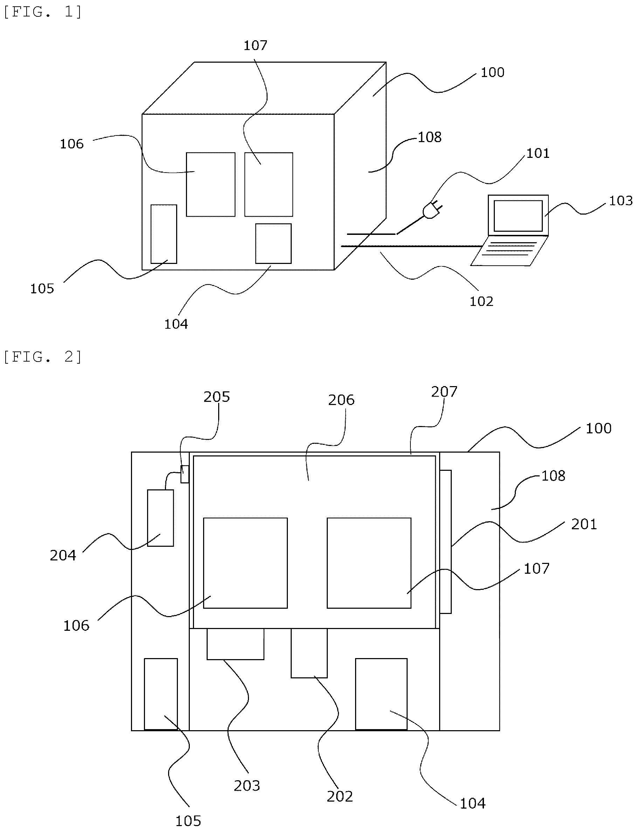

FIG. 1 is a diagram showing an overall configuration of a rapid microorganism testing device 100 including an optical measurement unit 202 (described later) that is an optical measurement device according to an embodiment. The rapid microorganism testing device 100 is a system including a control PC 103, and is supplied with power via a power cable 101. The rapid microorganism testing device 100 is connected to the control PC 103 via a communication cable 102.

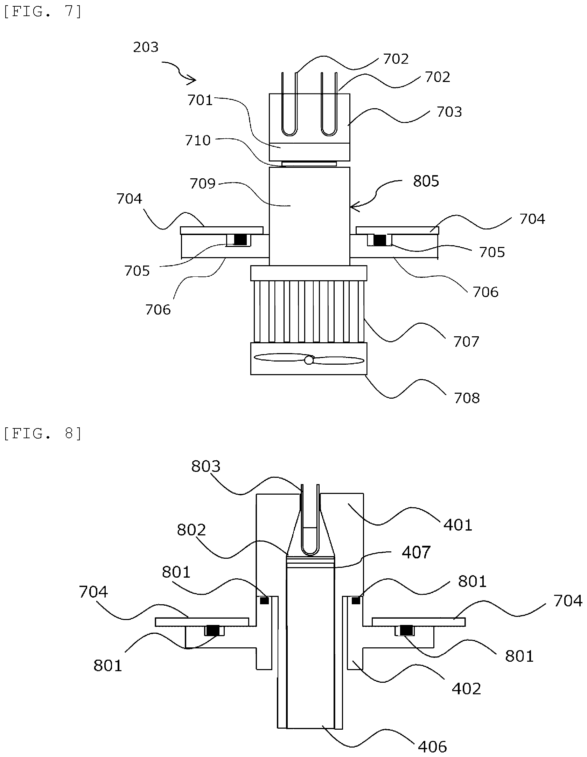

The rapid microorganism testing device 100 includes a system water drawer 105 in which system water for performing feeding and washing for a dispenser is contained, a waste liquid drawer 104 that accesses a waste liquid portion into which waste liquid for washing the dispenser flows, a reagent door 106 through which a luminescent reagent is provided on the rapid microorganism testing device 100, and a sample door 107 through which a sample tube 803 (shown in FIG. 8) is provided on the rapid microorganism testing device 100. Although not shown, each of the doors is an openable and closable door provided with a locking mechanism, or can be pulled out using a slide rail or the like.

FIG. 2 is a diagram showing a schematic configuration of the rapid microorganism testing device 100.

In FIG. 2, the rapid microorganism testing device 100 includes a dark box 207 whose inside is a dark box structure by a light-blocking structure, and a housing 108 surrounding a portion that is not the dark box structure. The dark box 207 includes a front panel 206 including the reagent door 106 and the sample door 107. The reagent door 106 and the sample door 107 are closed to form the light-blocking structure. The reagent door 106 and the sample door 107 are outside air intake portions that take in outside air.

Although not shown, a door that constitutes the light-blocking structure has a concave-convex structure and is black anodized at a door opening. A resin packing is sandwiched around an outer periphery of the front panel 206, thereby creating a structure that blocks light between the front panel 206 and the housing and forming the dark box 207.

Meanwhile, the system water drawer 105, the waste liquid drawer 104, a liquid feed syringe 204, a control board and a power supply necessary for an operation of the device, and the like are arranged outside the structure of the dark box 207. A pipe from the liquid feed syringe 204 to the inside is connected via a bulkhead pipe 205.

Although not shown, a harness connection to the dark box 207 is implemented by a light-blocking connection board 201, which is a board having a relay connector, and by sandwiching a packing for light blocking, a light-blocking connection is made, connecting a board and a power supply arranged outside the dark box structure to a unit arranged inside the dark box.

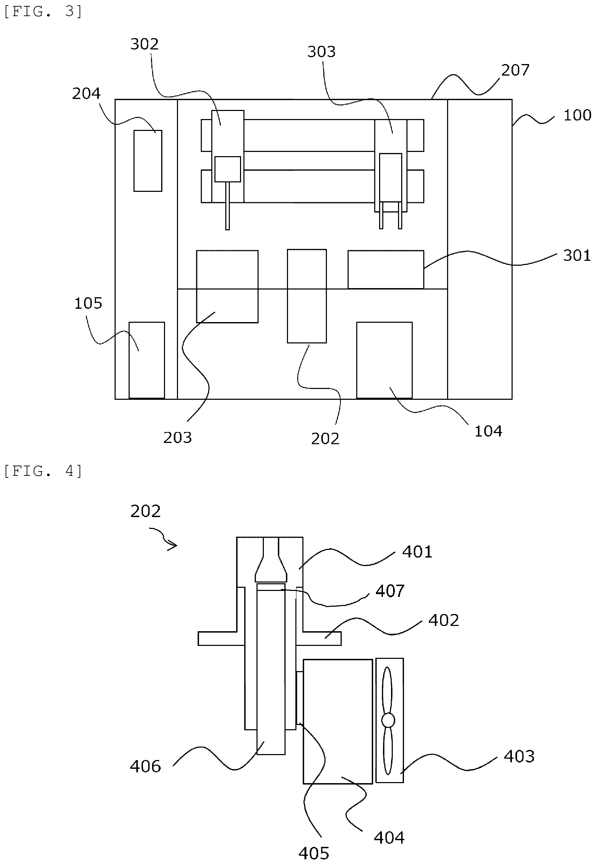

A reagent for a measurement is set in a reagent rack 203 through the reagent door 106. The reagent rack 203 includes a reagent temperature adjustment mechanism 805 as described later, and a temperature in the reagent rack 203 is adjusted to a constant temperature. The sample tube 803 is arranged in a tube rack, and is set in a sample rack mechanism 301 (shown in FIG. 3) through the sample door 107.

FIG. 3 is a diagram showing a schematic configuration of the inside of the rapid microorganism testing device 100.

In FIG. 3, the rapid microorganism testing device 100 includes the reagent rack 203 that stores a reagent, a photodetector unit 202 that performs a measurement, the sample rack mechanism 301 that holds the sample tube 803 accommodating a sample, a chuck arm 303 that conveys the tube rack inside the dark box 207, and a dispenser 302 that dispenses the reagent.

Generally, a detection can be performed with high sensitivity by using a photomultiplier tube (hereinafter referred to as PMT) as the photodetector unit 202.

However, when sensitivity as high as that of the PMT is not necessary, a semiconductor element such as a photodiode can be used. In this description, a case in which the PMT is used will be described.

The rapid microorganism testing device 100 includes the chuck arm 303 that conveys the sample tube 803 set in the sample rack mechanism 301.

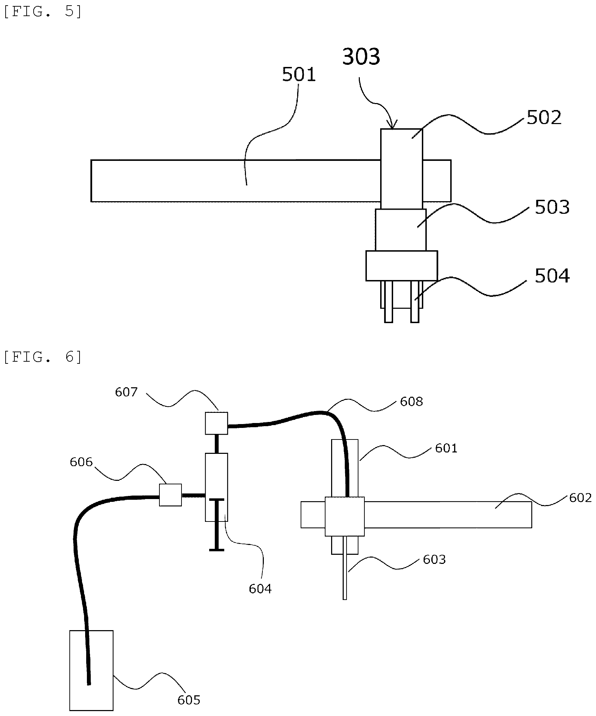

FIG. 5 is a schematic configuration diagram of a sample tube conveying mechanism. In FIG. 5, the chuck arm 303 grips the sample tube 803, and conveys a tube to be measured to the photodetector unit 202.

Although not shown, the sample rack mechanism 301 includes a Y drive unit capable of driving in a Y-axis direction, and these drive mechanisms make it possible to process a plurality of sample tubes 803. As the drive unit of the sample rack mechanism 301, for example, a drive mechanism using an actuator or a belt-pulley can be used.

The chuck arm 303 includes a tube griping portion 504 that grips the sample tube 803 and a chuck opening and closing mechanism 503 that opens and closes the tube griping portion 504. As the chuck opening and closing mechanism 503, for example, an opening and closing mechanism implemented by rotation of a rotary solenoid or an opening and closing mechanism using a push solenoid may be used.

Although not shown, the chuck gripping unit 504 includes two or more claws and is driven by the chuck opening and closing mechanism 503 to grip the sample tube 803. The chuck arm 303 includes an X drive unit 501 that can be driven in an X-axis direction and a Z drive unit 502 that can be driven in a Z direction, and conveys the sample tube 803 arranged in the tube rack to the photodetector unit 202 by being driven in the X direction and the Z direction. As a drive system of the chuck arm 303, for example, a drive mechanism using an actuator or a belt-pulley can be used.

FIG. 6 is a diagram showing a schematic configuration of a reagent dispenser.

In FIG. 6, suction of a reagent and discharge of the reagent to the sample tube 803 are performed by a dispensing nozzle 603. The dispensing nozzle 603 is connected to a syringe 604, an electromagnetic valve 606, an electromagnetic valve 607, and a system water 605 by a pipe 608. The dispensing nozzle 603 includes an X drive unit 602 that can be driven in the X-axis direction and a Z drive unit 601 that can be driven in the Z direction, and performs a dispensing operation of the reagent contained on the reagent rack 203 to the sample tube 803 arranged in the photodetector unit 202 by being driven in the X direction and the Z direction.

As a drive system, for example, a drive mechanism including an actuator or a belt-pulley may be used. Although the dispenser is not shown, the dispensing nozzle 603 moves to a cleaning port, and the inside and outside of the dispensing nozzle 603 are washed using the system water 605. A waste liquid after cleaning is discharged through a pipe to a waste liquid bottle provided in the waste liquid drawer 104 arranged outside the dark box 207.

FIG. 7 is a diagram showing a schematic configuration of the reagent rack 203.

In FIG. 7, the reagent rack 203 includes a rack 701 that holds a reagent holder 703 supporting a reagent tube 702, and the reagent temperature adjustment mechanism 805. A temperature of the rack 701 is adjusted to a constant temperature by the reagent temperature adjustment mechanism 805. FIG. 7 shows a configuration example of the temperature adjustment mechanism 805. The reagent temperature adjustment mechanism 805 includes a Peltier element 710, a heat sink 707, a heat dissipation block 709, and an air cooling fan 708.

The rack 701 of the reagent rack 203 is arranged in the dark box 207. Meanwhile, the heat sink 707 for heat dissipation and the air cooling fan 708 are arranged outside the structure of the dark box 207. A light-blocking block 706 is provided between the inside of the dark box 207 and the external heat sink 707, and blocks light to the dark box 207. The light-blocking block 706 includes a light-blocking member 705 and an intermediate plate 704 arranged inside the dark box 207.

FIG. 4 is a diagram showing a schematic configuration of the photodetector unit 202.

In FIG. 4, the photodetector unit 202 includes a detector 406, a temperature adjustment block 401 arranged in a manner of surrounding the detector 406, a light-blocking connection portion 402, and a sample temperature adjustment mechanism (a cooling fan 403, a heat sink 404, and a Peltier element 405).

A light-receiving unit 407 of the detector 406 is arranged in the dark box 207 in a manner of facing the sample tube 803, and the sample temperature adjustment mechanism is arranged outside the structure of the dark box 207. A configuration example of the sample temperature adjustment mechanism is shown in FIG. 4. The sample temperature adjustment mechanism includes the Peltier element 405, the heat sink 404, and the air cooling fan 403, and controls the temperature adjustment block 401 surrounding the detector 406 to a constant temperature.

The dark box 207 is surrounded by the housing 108 having an outside air intake portion, and the temperature adjustment block 401 is arranged across the dark box 207 and the inside of the housing 108 which is outside the dark box 207.

FIG. 8 is a diagram schematically showing a light-blocking structure in the photodetector unit 202.

In FIG. 8, the light-blocking connection portion 402 has a light-blocking structure in which a light-blocking member 801 is sandwiched in an opening formed in the intermediate plate 704 constituting the dark box 207. A connection portion between the light-blocking connection portion 402 and the temperature adjustment block 401 similarly has a light-blocking structure in which the light-blocking member 801 is sandwiched. The light-blocking member 801 may be made of a resin material, a packing, or the like. In addition to the structure in which the light-blocking member 801 is sandwiched, it is also possible to perform light blocking by providing a concave-convex structure at a portion in which light-blocking connection is performed.

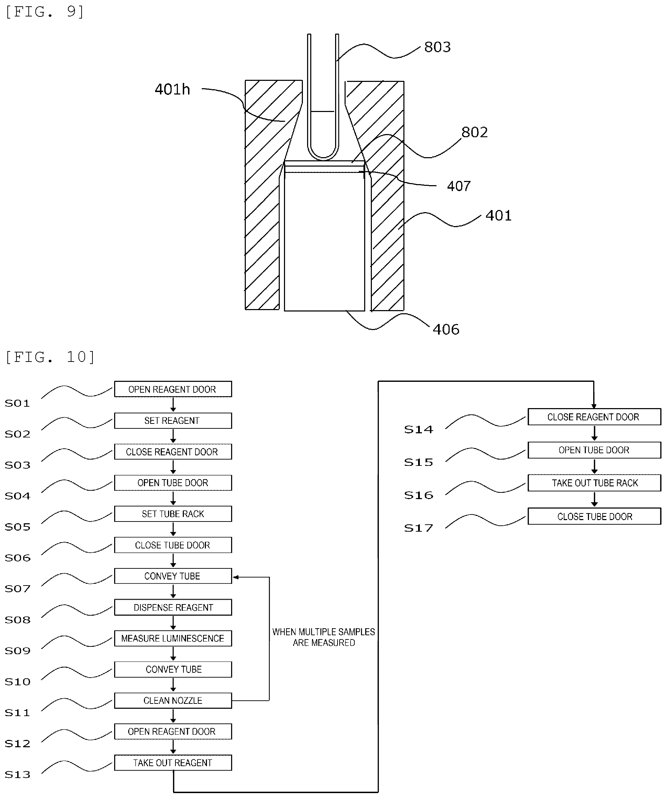

FIG. 9 shows a schematic configuration of the light-receiving unit of the photodetector unit 202. The temperature adjustment block 401 includes a sample tube holding portion 401h that holds the sample tube 803. As shown in FIG. 9, a conical internal space (conical portion) is formed in the sample tube holding portion 401h, and the sample tube 803 is held in the internal space. By holding the sample tube 803 in the internal space, stabilization of a temperature of the sample accommodated in the sample tube 803 is improved.

An inner wall of the conical portion of the sample tube holding portion 401h uses specular reflection to collect light scattered in a direction different from that in the light-receiving unit of the detector 406. In order to implement the specular reflection, a member obtained by polishing or plating a metal material is used. It is possible to obtain stable reflection efficiency by using aluminum or the like as the metal material.

A shape of the portion holding the tube is not limited to the conical shape, but may be another shape such as a semicircular shape.

As shown in FIG. 9, the sample tube 803 arranged on the detector 406 is arranged above the light-receiving unit 407 of the photodetector unit 202 that performs detection. The shorter a distance between the sample tube 803 and the light-receiving unit 407 of the detector 406 is, the higher detection sensitivity is. However, the sample tube 803 is often made of plastic, and may be electrostatically charged.

Therefore, discharge may occur when the sample tube 803 and the light-receiving unit 407 are brought close to each other. The detector 406 may not be able to perform an accurate measurement due to the discharge or the like.

Therefore, a glass plate 802 is arranged between the detector 406 and the sample tube 803. The glass plate 802 is made of a material such as a quartz plate that has sufficient transmittance in a visible region. It is also possible to select a fluorescent filter or the like having a specific band in a wavelength range of a luminescence reaction.

FIG. 10 is a flowchart showing a typical example of a luminescence measurement in one embodiment of the invention.

In FIG. 10, after the rapid microorganism testing device 100 and software in the control PC 103 are started, the reagent door 106 is opened (step S01), and the reagent holder 703 in which the luminescent reagent is set is provided in the rack 701 (step S02). After the reagent holder 703 is set in the rack 701, the reagent door 106 is closed (step S03).

Next, the sample door 107 is opened (step S04), and the tube rack in which a tube containing a measurement sample is set is provided in the sample rack mechanism 301 (step S05). After the tube rack is provided in the sample rack mechanism 301, the sample door 107 is closed (step S06). A measurement condition is input by control software, and a measurement start button of the control PC 103 is pressed.

After the measurement is started, the chuck arm 303 conveys the sample tube 803 from the sample rack mechanism 301 to the photodetector unit 202 (step S07). The luminescent reagent is dispensed, by the dispenser 302, from the reagent tube 702 set in the reagent holder 703 into the conveyed sample tube 803 (step S08).

After the reagent is dispensed into the sample tube 803, luminescence occurs and a luminescence amount is measured by the detector 406 (step S09).

After the luminescence amount is measured, the sample tube 803 is conveyed to the sample rack mechanism 301 by the chuck arm 303 (step S10). The dispenser 302 moves to the cleaning port, and the dispensing nozzle 603 is cleaned using the system water 605 (step S11). When there are a plurality of samples, the sample tube 803 to be measured next is conveyed, and a luminescence measurement is sequentially performed (steps S07 to S11).

After all the samples are measured, the reagent door 106 is opened (step S12), and the luminescent reagent is taken out (step S13). After the luminescent reagent is taken out, the reagent door 106 is closed (step S14) and the sample door 107 is opened (step S15). The sample tube rack is taken out (step S16), and then the sample door 107 is closed (step S17).

Acquired data on the luminescence amount is sent to the control PC 103, and conversion from the luminescence amount to an ATP concentration is performed using a calibration curve created in advance in the control PC 103. A plurality of calibration curves exist in the software according to the invention. The conversion to the ATP concentration is performed using a selected calibration curve, and measurement data and calibration curve information are stored in association with each other.

As described above, the optical measurement device 202 according to the embodiment of the invention includes: the detector 406 that includes the light-receiving unit 407 receiving light generated from the sample tube 803 arranged in the dark box 207 and that detects the generated light; the temperature adjustment block 401 that is arranged around the detector 406, a part of the temperature adjustment block 401 being arranged in the dark box 207, and the other part of the temperature adjustment block 401 being arranged outside the dark box 207; and the temperature adjustment mechanisms 403, 404, 405 that are connected to the portion of the temperature adjustment block 401 arranged outside the dark box 207.

A part of the temperature adjustment block 401 covers the light-receiving unit 407 of the photodetector unit 202 in the dark box 207, and the other part of the temperature adjustment block 401 is arranged outside the dark box 207. A part of the temperature adjustment block 401 arranged outside the dark box 207 is provided with the cooling fan 403, the heat sink 404, and the heat sink 404 which are the temperature adjustment mechanisms.

Thus, according to the embodiment of the invention, it is possible to implement an optical measurement device capable of reducing influence of heat dissipation from a temperature adjustment mechanism that adjusts a temperature in the dark box 207 in which a sample is arranged, efficiently maintaining a low temperature inside the dark box 207 while maintaining a light-blocking effect, and detecting bioluminescence and chemiluminescence of a substance with high sensitivity and high precision.

According to the embodiment of the invention, the Peltier element 710 that keeps the temperature of the rack 701 holding the reagent holder of the reagent tube 702 constant is arranged in the dark box 207, and the heat sink 707 and the cooling fan 708 that are connected to the heat dissipation block 709 of the Peltier element 710 are arranged outside the dark box 207, and thus a reagent in the dark box 207 can be efficiently maintained at a constant temperature.

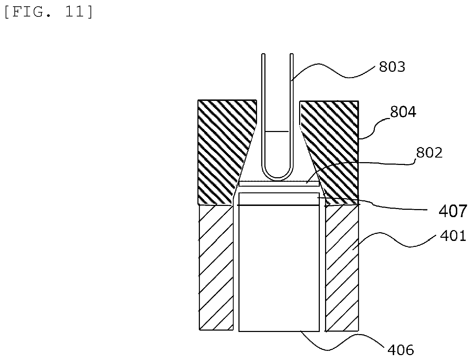

In the above embodiment, as shown in FIGS. 4, 8, and 9, the light-receiving unit 407 of the photodetector unit 202 is surrounded by the temperature adjustment block 401. Alternatively, as shown in FIG. 11, the temperature adjustment block 401 may be configured not to surround the light-receiving unit 407.

In a modification shown in FIG. 11, the light-receiving unit 407 is surrounded by a sample tube support member 804, which supports the glass plate 802 and also holds the sample tube 803. FIG. 9 shows a schematic configuration of the light-receiving unit of the optical measurement device. The temperature adjustment block 401 includes the sample tube holding portion 401h that holds the sample tube 803.

In an example shown in FIG. 11, similarly to the example shown in FIG. 9, a conical internal space (conical portion) is formed in the sample tube support member 804, and the sample tube 803 is held in the internal space. By holding the sample tube 803 in the internal space, the stabilization of the temperature of the sample accommodated in the sample tube 803 is improved.

### Reference Signs List

- 100:: rapid microorganism testing device
- 101:: power cable
- 102:: communication cable
- 103:: control PC
- 104:: waste liquid drawer
- 105:: system water drawer
- 106:: reagent door (outside air intake portion)
- 107:: sample door (outside air intake portion)
- 108:: housing
- 201:: light-blocking connection board
- 202:: photodetector unit (optical measurement device)
- 203:: reagent rack
- 204:: liquid feed syringe
- 205:: bulkhead pipe
- 206:: front panel
- 207:: dark box
- 301:: sample rack mechanism
- 302:: dispenser
- 303:: chuck arm
- 401:: temperature adjustment block
- 401h:: sample tube holding portion
- 402:: light-blocking connection portion
- 403:: air cooling fan
- 404:: heat sink
- 405:: Peltier element
- 406:: detector
- 407:: light-receiving unit
- 501:: X drive unit
- 502:: Z drive unit
- 503:: chuck opening and closing mechanism
- 504:: tube griping portion
- 601:: Z drive unit
- 602:: X drive unit
- 603:: dispensing nozzle
- 604:: syringe
- 605:: system water
- 606:: electromagnetic valve 1
- 607:: electromagnetic valve 2
- 608:: pipe
- 701: : rack
- 702:: reagent tube
- 703:: reagent holder
- 704:: intermediate plate
- 705:: light-blocking member
- 706:: light-blocking block
- 707:: heat sink
- 708:: air cooling fan
- 709:: heat dissipation block
- 710:: Peltier element
- 801:: light-blocking member
- 802:: glass plate
- 803:: sample tube
- 804:: sample tube support member
- 805:: reagent temperature adjustment mechanism

## Claims

1. An optical measurement device comprising:
a detector that includes a light-receiving unit configured to receive light generated from a sample tube arranged in a dark box and that is configured to detect the generated light;
a temperature adjustment block arranged around the detector; and
a sample temperature adjustment mechanism connected to the temperature adjustment block, wherein
a part of the temperature adjustment block is arranged in the dark box and the other part of the temperature adjustment block is arranged outside the dark box, and the sample temperature adjustment mechanism is connected to a portion of the temperature adjustment block arranged outside the dark box.

2. The optical measurement device according to claim 1, wherein
the temperature adjustment block is arranged such that an outer peripheral portion of the light-receiving unit is covered in the dark box.

3. The optical measurement device according to claim 2, wherein
the temperature adjustment block includes a sample tube holding portion that holds the sample tube in the dark box.

4. The optical measurement device according to claim 1, wherein
the dark box is surrounded by a housing having an outside air intake portion, and the temperature adjustment block is arranged across the dark box and the inside of the housing which is outside the dark box.

5. The optical measurement device according to claim 1, wherein
a mechanism configured to convey the sample tube and a dispensing mechanism configured to dispense a reagent are arranged inside the dark box.

6. The optical measurement device according to claim 1, wherein
the dark box is provided with a reagent temperature adjustment mechanism, a part of which is arranged in the dark box and the other part of which is arranged outside the dark box, and
the reagent temperature adjustment mechanism includes a Peltier element, a heat sink, a heat dissipation block, and a cooling fan, the heat sink and the cooling fan being arranged outside the dark box.
